# FASCICULE DE BREVET EUROPEEN

(11) **EP 1 714 658 B1**
(45) Date de publication et mention de la délivrance du brevet: **21.01.2009**
(21) Numéro de dépôt: 06112921.9
(22) Date de dépôt: 21.04.2006
(51) Int. Cl.: A61K 33/30, A61K 33/04, A61K 33/06, A61K 31/375, A61K 38/06, A61K 38/40, A61K 31/205, A61P 15/08, A61P 15/06

(54) **Complément alimentaire comprenant une association d'agents antioxydants et de la carnitine ou un précurseur de la carnitine, comme aide à la fertilité chez l'homme et chez la femme**
Nahrungsengänzungsmittel, enthaltend antioxidative Wirkstoffe und Karnitin oder ein Vorprodukt davon, zur Verbesserung der Fertilität bei Männer und Frauen
Dietary supplement comprising antioxidant agents and carnitine or a precursor thereof, to be used as an aid to fertility in men and women

(30) Priorité: 21.04.2005 FR 0503998
(43) Date de publication de la demande: 25.10.2006
(73) Titulaire: Formquest Limited, London N1 4BY (GB)
(72) Inventeur: Cohen, Marc, 69006 Lyon (FR)
(74) Mandataire: Collin, Jérôme

(56) Documents cités:
- EP-A- 1 163 904
- EP-A- 1 195 159
- WO-A-03/086080
- US-A- 6 080 788
- US-B1- 6 576 634
- SINCLAIR S: "MALE INFERTILITY: NUTRITIONAL AND ENVIRONMENTAL CONSIDERATIONS" ALTERNATIVE MEDICINE REVIEW, THORNE RESEARCH INC., SANDPOINT,, US, vol. 5, no. 1, 2000, pages 28-38, XP008014042 ISSN: 1089-5159

## Description

L'invention concerne de nouveaux compléments alimentaires qui sont utiles comme aide à la fertilité chez l'homme et chez la femme.

Les causes d'une infertilité se recherchent à quatre niveaux : qualité du sperme, ovulations, voies génitales féminines et masculines, et incompatibilité entre le sperme et le milieu génital féminin.

De nombreuses études ont permis de mettre en évidence le déclin de la qualité du sperme des hommes fertiles, depuis une vingtaine d'années, ce qui entraînerait une altération de la fécondité dans les années à venir.

La question de l'infertilité conjugale doit faire prendre conscience de l'incidence sur la fertilité masculine et féminine des facteurs liés à l'environnement et des facteurs nutritionnels.

Dans les pays industriels, 15 % des couples sont inféconds dont les causes identifiées sont diverses mais une grande proportion ne montre aucune anomalie au niveau de chacun des deux conjoints et cependant ils n'arrivent pas à concevoir un enfant : c'est l'infécondité inexpliquée.

L'infécondité peut avoir pour origine l'action des agents toxiques, pesticides (dioxine avec effet oestrogénique par blocage des récepteurs aux androgènes), les métaux lourds, le tabac (par le biais des radicaux libres), l'alcool, les agents pharmacologiques, les rayons X et gamma, les hormones (absorbées par le bétail puis consommées ensuite par les humains), l'infection ou l'inflammation. Ces facteurs sont susceptibles d'entraîner l'apparition d'un stress oxydatif. Le stress oxydatif est, dans ce contexte, l'intermédiaire dans l'infécondité.

Explication du stress oxydatif : 98% de l'oxygène inspiré est réduit en molécules d'eau par capture de quatre électrons et s'accompagne de formation d'A.T.P., qui est à l'origine de la production d'énergie par la cellule pour sa défense, sa mobilité, sa reproduction. Mais dans 2% de cas, il y a une fuite d'électrons, qui entrent directement en rapport avec l'oxygène. Cet oxygène va être affecté d'un électron célibataire, ce qui va aboutir à la formation d'espèces oxygénées réactives toxiques. C'est ainsi que naissent :
- l'anion superoxyde ;
- le peroxyde d'hydrogène ;
- l'acide péroxynitrique ; et
- l'oxygène singulet.

Le stress oxydatif peut s'aggraver à l'occasion de certaines situations : pollution, xénobiotiques, surexposition au soleil, irradiations, conditions infectieuses, inflammatoires ou dégénératives. D'autre part, le vieillissement s'accompagne d'une altération du transport des électrons et donc d'une augmentation des espèces oxygénées réactives.

En réponse à la formation d'espèces oxygénées, notre organisme a développé des lignes de défense nous protégeant des effets nocifs des espèces oxygénées réactives par combinaison d'agent antioxydants avec les radicaux libres pour former des dérivés oxydés neutres.

Le stress oxydatif résulte du déséquilibre entre les espèces oxygénées destructrices et les antioxydants protecteurs (espèces oxygénées destructrices > antioxydants protecteurs).

Les conséquences possibles du stress oxydatif sur la fécondité sont les suivantes :
1) Cassures au niveau des chromosomes altérant le message génétique (mesuré par l'index de fragmentation de l'ADN du spermatozoïde (DFI)) ;
2) Péroxydation des acides gras polyinsaturés des membranes cellulaires entraînant un déficit de la perméabilité et des troubles dans les échanges pouvant aller jusqu'à la mort cellulaire.

Chez l'homme, le stress oxydatif va affecter:
- la concentration du sperme (oligospermie > azoospermie)
- la mobilité des spermatozoïdes (asthénospermie, moins de 40 % de spermatozoïdes mobiles)
- la morphologie des spermatozoïdes (tératospermie, moins de 30 % de spermatozoïdes normaux)

Chez la femme, le stress oxydatif peut entraîner sur l'ovocyte les mêmes inconvénients que pour le spermatozoïde (attaque de l'ADN et des acides gras polyinsaturés de la membrane). En outre, la femme est exposée à des risques spécifiques, selon le déficit en certains antioxydants. C'est ainsi que le déficit de sélénium (qui s'accompagne d'une baisse du glutathion peroxydase) pourrait entraîner une fausse couche par suite des dégâts oxydatifs directs sur les membranes et l'ADN du foetus. Le déficit en vitamines B6, B9 et B12 peut également entraîner une fausse couche, cette fois par le biais de l'élévation de l'homocystéine (dégâts vasculaires précoces au niveau de la nidation). Le risque de fausse couche peut de nouveau être amplifié lorsqu'il y a un déficit en zinc (répercussion sur l'ovogenèse). Enfm, le déficit en vitamines B6 et B9 et en zinc peut entraîner chez le foetus des malformations du tube neural (spina bifida) ainsi que des malformations vésicales et cardiaques.

La demande de brevet internationale WO 03/086080 décrit une composition pharmaceutique et un complément alimentaire, ayant une activité de promotion de la fertilité chez l'homme (sexe masculin), qui comprend des vitamines C et E, du sélénium, de l'acide ferulique, du zinc, des vitamines du groupe B, et éventuellement la L-carnitine et la co-enzyme Q10. Des nutriments tels que la L-carnitine, les vitamines du groupe B et le zinc jouent un rôle dans le métabolisme et la formation du sperme. Les vitamines C et E, le thé vert, avec ou sans co-enzyme Q10, et le sélénium sont des agents anti-oxydants qui permettent l'amélioration de la qualité et de la quantité du sperme.

Des études sur les causes d'infertilité et leurs traitements ont déjà été réalisées ; elles sont résumées dans l'article S. Sinclar, Alternative Medicine Review, pages 28-38, vol 5, n°1, 2000. D'après cette synthèse, il est connu que la carnitine, le zinc, la vitamine E, la co-enzyme Q10 et la vitamine B12 agissent sur la motilité du sperme.

La carnitine agit également sur la maturité du sperme.

Le zinc, la co-enzyme Q10 et la vitamine B12 agissent également sur la production du sperme (des études supplémentaires pour la co-enzyme Q10 sont nécessaires).

En outre, il est connu que les acides gras polyinsaturés protègent les membranes cellulaires des spermatozoïdes et permettent de prévenir le stress oxydatif.

La vitamine C permet de protéger le partimoine génétique du sperme. Le glutathion et le sélénium sont deux éléments essentiels pour la défense antioxydante du sperme, le glatathion, mais non le sélénium, permet également d'augmenter la motilité du sperme.

Jusqu'à présent, ces différents traitements ont été utilisés isolément. De plus, la cible du traitement de l'infertilité est préférentiellement la motilité du sperme.

Or, l'inventeur a découvert qu'il était plus efficace de rechercher une protection globale du spermatozoïde en agissant simultanément sur
- la protection des membranes ;
- la protection du bagage génétique ;
- la motilité et la trajectoire ;
- l'apport d'énergie.

Cette protection globale permet une amélioration significative des compétences des spermatozoïdes.

En effet, il n'est pas suffisant de rechercher uniquement une amélioration de la motilité des spermatozoïdes alors que les causes de l'infertilité sont multifactorielles, le stress oxydatif pouvant frapper de manière aléatoire les différentes parties du spermatozoïde.

La présente invention vise à résoudre ce nouveau problème technique (la protection du spermatozoïde dans sa globalité) et propose un nouveau complément alimentaire.

Dans ce complément alimentaire, l'augmentation et la diversification des agents antioxydants associés permet une protection globale du spermatozoïde.

L'invention a pour objet un complément alimentaire qui permet de lutter contre le stress oxydatif et ainsi d'aider à la fertilité masculine ou féminine, et de réduire les risques de fausse couche et de malformation.

L'invention concerne donc un complément alimentaire, destiné à une administration par voie orale, comprenant une association :
(a) d'un mélange d'agents antioxydants et énergisants constitué des vitamines C et E, du co-enzyme Q10 et/ou d'une association de vitamines B2, B5, B6, B9, B 12 et C, du zinc (sous forme de sels ou de complexes), du glutathion ou de la lactoferrine, de la superoxyde dismutase, du sélénium et d'acides gras insaturés ;
(b) d'un mélange d'agents énergisants constitué de la carnitine et/ou un produit capable de générer la carnitine par synthèse endogène, et du magnésium.

La vitamine C permet de protéger l'ADN des radicaux libres et de protéger les hydroxylases indispensables à la production d'énergie.

Selon une caractéristique particulière de la présente invention, le complément alimentaire comprend 60 à 120 mg de vitamine C (vitamine C naturelle).

La vitamine E permet de protéger la membrane lipidique contre la lipopéroxydation et contribue ainsi à la stabilisation membranaire. Selon une caractéristique particulière de la présente invention, le complément alimentaire comprend 3 à 6,5 mg de vitamine E.

Le co-enzyme Q10 (COE Q10) est un puissant antioxydant mitochondrial, intermédiaire de la chaîne respiratoire mitochondriale. Il est un élément important pour l'énergie des spermatozoïdes.

Le co-enzyme Q10 peut être introduit tel que ou également être apporté par synthèse endogène et/ou par voie exogène. Ainsi, l'association de vitamines B2, B5, B6, B9, B12 et C permet la synthèse endogène du co-enzyme Q10. D'autre part, la source de co-enzyme Q10 peut être un extrait naturel, avantageusement une huile de soja. Cette huile de soja est avantageusement introduite dans le complément alimentaire en une quantité variant de 50 à 100 mg.

Selon une variante préférée de l'invention, le co-enzyme Q10 est introduit dans le complément alimentaire par apport endogène et exogène : le complément alimentaire selon l'invention comprend alors une association de vitamines B2, B5, B6, B9, B12 et C et de l'huile de soja (avantageusement 50 à 100 mg d'huile de soja).

Le zinc joue un rôle dans le développement testiculaire, notamment dans la transformation de la testostérone en DHT (dihydrotestostérone, forme active de la testostérone). Le zinc se présente avantageusement sous forme de sel de zinc, tel que le citrate de zinc, ou sous forme d'un complexe. Dans une variante avantageuse de l'invention, le zinc est introduit sous la forme de citrate de zinc. Le complément alimentaire selon l'invention comprend alors avantageusement 12 à 25 mg de citrate de zinc.

Le glutathion est une protéine que l'organisme humain produit naturellement et qui est composée de trois acides aminés (cystéine, acide glutamique et glycine). C'est un agent antioxydant et énergisant, qui nécessite la présence d'une part d'un co-facteur, la superoxyde dismutase, et d'autre part, pour que son activité soit complète, de sélénium. Le glutathion permet d'assurer l'intégrité du flagelle du spermatozoïde.

Dans le complément alimentaire selon l'invention, on utilise avantageusement un générateur du glutathion : la lactoferrine. En particulier, on utilise un extrait de petit lait (source de lactoferrine), avantageusement 50 à 100 mg d'extrait de petit lait.

Le superoxyde dismutase (SOD) est une enzyme qui élimine les radicaux hydroxyles et bloque ainsi le processus de formation des radicaux libres dès son début. La SOD peut être d'origine animale (bovine par exemple) ou végétale. En particulier, la SOD est présente dans le melon (cucumis melo).

Dans le complément alimentaire selon l'invention, on utilise avantageusement, en tant que source de superoxyde dismutase, un extrait de melon, plus particulièrement 2 à 5 mg d'extrait de melon.

Le sélénium joue un rôle dans la biosynthèse de la testostérone et donc dans la spermatogenèse. Il permet de préserver l'intégrité du flagelle. Il est avantageusement introduit dans le complément alimentaire sous la forme de sélénite de sodium. Le complément alimentaire selon l'invention comprend alors avantageusement 0,042 à 0,084 mg de sélénite de sodium.

Les acides gras insaturés (ou graisses insaturées) ont des effets significatifs sur la qualité fonctionnelle des membranes et permettent d'assurer la survie des spermatozoïdes (les membranes des spermatozoïdes sont constituées de graisses insaturées). On préfère les acides gras insaturés à longue chaîne, en particulier les acides gras en ω-6. Dans le complément alimentaire selon l'invention, on utilise avantageusement l'huile de bourrache et/ou l'huile d'onagre, en particulier 25 à 50 mg d'huile de bourrache et/ou 25 à 50 mg d'huile d'onagre.

Ce mélange (a) d'agents à la fois antioxydants et énergisants permet la protection des structures des gamètes et assure également la production d'énergie, dont les gamètes font une grande consommation.

Le mélange (b) d'agents purement énergisants, assure principalement la production énergétique pour les gamètes.

La carnitine permet d'assurer le transport des acides gras sur leur site d'oxydation en vue de la production d'énergie. Elle peut être introduite dans le complément alimentaire selon l'invention telle quelle ou par apport endogène ou exogène. On préfère un apport exogène combiné à une synthèse endogène.

Dans le cadre de la présente invention, l'apport exogène de carnitine est avantageusement un extrait naturel, en particulier la spiruline (contient de la lysine et de la méthionine). Le complément alimentaire selon l'invention contient ainsi, de préférence, 25 à 50 mg de spiruline.

Dans le cadre de la présente invention, l'apport par synthèse endogène de carnitine est avantageusement obtenu par l'utilisation d'une association de vitamines B3 et C. La vitamine B3 permet en outre la protection des hydroxylases. Le complément alimentaire contient avantageusement de 3 à 7 mg de vitamine B3.

L'action de la carnitine, obtenue par un apport exogène ou par synthèse endogène, peut être renforcée par l'action des vitamines B3, B6 et B9.

Le magnésium est un élément énergisant. Il permet de lutter contre l'asthénie des spermatozoïdes. Il est avantageusement introduit dans le complément alimentaire sous la forme d'oxyde de magnésium (apport exogène). Le complément alimentaire selon l'invention comprend alors avantageusement 80 à 120 mg d'oxyde de magnésium.

Le complément alimentaire selon l'invention peut également comprendre un troisième mélange (c) comprenant au moins un agent anti-oxydant choisi dans le groupe constitué par les catéchines, l'hydroxytyrosol, les flavonoïdes, la béta-carotène et leurs mélanges. Ce mélange (c) permet de renforcer l'action antioxydante du mélange (a).

Le complément alimentaire selon l'invention peut également comprendre un quatrième mélange (d), comprenant au moins un agent anti-oxydant choisi dans le groupe constitué par les OPC de pin (picnogénol) et la xéaxantine. Ce mélange (d) permet d'accentuer la baisse du DFI, ce qui signifie une meilleure protection de l'ADN.

On constate que de plus en plus de couples rencontrent des difficultés pour concevoir un enfant.

L'infertilité masculine est multifactorielle :
- anomalies génétiques (translocations, délétion du chromosome y ...)
- anticorps antispermatozoides, Varicocoeles...
- impact de l'environnement (désordres endocriniens avec xénoestrogènes et antiandrogènes (produits toxiques avec le plomb, cadmium...))
- mode de vie : alimentation déséquilibrée (obésité, tabagisme, alcool, drogue)

L'intervention nutritionnelle ne peut concerner que le mode vie et l'impact de l'environnement avec pour objectif principal le diagnostic de l'importance du stress oxydant dont l'impact sur les spermatozoïdes peut être très divers du fait qu'il peut toucher toutes les structures du spermatozoïde : protéiques, graisses insaturées etc..

L'expression de ce dysfonctionnement peut être
- une atteinte de la production des spermatozoïdes ;
- une atteinte de leur motilité et de leur trajectoire ;
- une atteinte de leur intégrité ;
- une atteinte de l'ADN (identifiée par le test de fragmentation de l'ADN) ;
- une oxydation des graisses insaturées (identifiée par l'Analyse des phospholipides des membranes).

Les atteintes de la production des spermatozoïdes, de leur motilité et de leur trajectoire et de leur intégrité sont identifiées par le spermogramme et spermocytogramme.

Il a été constaté par l'inventeur que la prévention de ces dysfonctionnements par un supplément alimentaire ne peut être que multifactorielle.

En effet, l'inventeur a déterminé que lorsqu'on utilise uniquement les antioxydants conventionnels (Vitamines A, C, E, et Sélénium) l'impact est inconstant :
- sur la survie et la motilité des spermatozoïdes (comme l'atteste les résultats de l'étude des spermatozoïdes soumis à une coloration éosine -Négrosine),
- sur le DFI,
- et sur l'analyse des phospholipides membranaires.

Ainsi, l'utilisation seule de ces antioxydants n'est pas suffisante pour assurer une protection des spermatozoïdes.

L'inventeur a ensuite montré que lorsqu'on utilise, outre les antioxydants classiques, des antioxydants tels que la L-carnitine ou acétyl Carnitine et le COE Q10 on obtient une augmentation substantielle de la motilité et de la production des spermatozoides et une baisse du DFI ce qui signifie une meilleure protection de l'ADN. (résultats obtenus lors d'une étude en double aveugle avec placebo).

Ce phénomène est amplifié si on ajoute des acides gras polyinsaturés (AGPI) notamment à longue chaîne (oméga 6), et/ou si on ajoute des OPC de pin et de la Xéaxantine. L'amélioration porte surtout sur le DFI. Les OPC de pin et la xéaxantine ont une action sur l'oxygène singulet, acteur privilégié de l'oxydation des protéines des spermatozoïdes.

En conséquence, la formulation des antioxydants pour la stérilité doit tenir compte de tous ces faits : l'impact aléatoire de l'agression radicalaire doit être combattue par différents groupes d'antioxydants complémentaires ayant des spécifités d'action sur les différentes cibles du spermatozoïde avec cet élément essentiel que constitue les AGPI, notamment à longue chaînes (oméga 6).

L'invention concerne ainsi l'utilisation du complément alimentaire selon l'invention dans le traitement de l'infertilité (masculine ou féminine), notamment de l'infertilité conjugale. Le complément alimentaire selon l'invention est efficace pour l'aide à la fertilité, en raison notamment de son rôle préventif envers le stress oxydatif.

L'invention concerne également l'utilisation du complément alimentaire selon l'invention dans la prévention des fausses couches, en particulier des fausses couches précoces, et des malformations embryonnaires.

Le complément alimentaire selon l'invention permet d'assurer la protection des spermatozoïdes et des ovocytes. De plus, il permet de prévenir les dégâts oxydatifs (sur les membranes, sur l'ADN) sur l'embryon et diminue ainsi le risque de fausses couches, notamment de fausses couches précoces. Le complément selon l'invention permet également de prévenir les risques de dégâts vasculaires précoces au niveau de la nidation (empêcher une élévation de l'homocystéine) et ainsi il permet également, par le biais de ce deuxième mécanisme, de diminuer le risque de fausses couches, notamment de fausses couches précoces.

Ainsi, ledit complément permet avantageusement de réduire les risques de fausses couches précoces et les risques de malformations embryonnaires, notamment les malformations :
- du tube neural ;
- vésicales ;
- cardiaques.

Les différents composés du complément alimentaires peuvent être administrés simultanément (dans un même comprimé par exemple) ou comme produits de combinaison pour une utilisation séparée ou étalée dans le temps. Ils sont de préférence administrés simultanément.

Le complément alimentaire est formulé pour être administré par voie orale. Il peut ainsi se présenter sous forme de capsule, gélule, comprimé, granule, avantageusement sous la forme de comprimé. Lorsque le complément alimentaire se présente sous forme de capsule molle ou de gélule, l'enveloppe de ces capsules molles ou de ces gélules peut contenir notamment de la gélatine animale telle que la gélatine de poisson, de la glycérine, ou un matériau d'origine végétale tel qu'un dérivé de cellulose ou d'amidon, ou une protéine végétale. Lorsque le complément alimentaire se présente sous forme de gélule, de comprimé, ou de granule, le mélange d'actifs peut être fixé sur un support pulvérulent tel que la silice, la cellulose, et la maltodextrine.

Les conseils d'utilisation sont de deux doses par jour pour le père et/ou de deux doses par jour pour la mère.

Avantageusement selon la présente invention, le complément alimentaire peut comprendre tout véhicule ou excipient approprié, acceptable du point de vue nutraceutique, ainsi que des additifs conventionnels, connus de l'homme du métier.

Le complément alimentaire selon l'invention répond de préférence à la composition pondérale suivante :

Pour 2 comprimés de 769,48 mg, par comprimé :

| | | |
|---|---|---|
| Vitamine C naturelle | 60 - 120 mg | |
| Oxyde de magnésium | 80 - 120 mg | |
| Extrait de petit lait | 50 - 100 mg | |
| Huile de soja poudre | 50 - 100 mg | |
| Huile bourrache poudre | 25 - 50 mg | |
| Huile d'onagre poudre | 25 - 50 mg | |
| Spiruline poudre | 25 - 50 mg | |
| Citrate de zinc | 12 - 25 mg | |
| Alpha tocophérol | 3 - 6,50 mg | |
| Riboflavine | 0,40 - 0,80 mg | (vitamine B2) |
| Nicotinamide | 3 - 7 mg | (vitamine B3) |
| Pantothénate de calcium | 2 - 3,25 mg | (vitamine B5) |
| Chlorhydrate de pyridoxine | 0,5 - 1 mg | (vitamine B6) |
| Acide folique | 0,05 - 1 mg | (vitamine B9) |
| Cyanocobalamine | 0,00060 - 0,000120mg | (vitamine B12) |
| Sélénite de sodium | 0,042 - 0,084 mg | |
| Extrait de thé vert | 20 - 50 mg | |
| Extrait d'olive fruit | 20 - 35 mg | |
| Extrait de citrus | 10 - 25 mg | |
| Bêta carotène naturel | 10 - 20 mg | |
| Extrait de melon | 2 - 5 mg | |
| Extrait de tomate | 0,5 - 1 mg | |

Pour ce comprimé particulier, la dose recommandée est de deux comprimés par jour.

Selon une variante avantageuse, le complément alimentaire répond à la composition pondérale suivante :

| | | |
|---|---|---|
| Pour 2 comprimés de l'odre de 750 mg, par comprimé : | | |
| Vitamine C naturelle | 60 - 120 mg | |
| Oxyde de magnésium | 80 - 120 mg | |
| Extrait de petit lait | 50 - 100 mg | |
| Huile de soja poudre | 50 - 100 mg | |
| Huile bourrache poudre | 25 - 50 mg | |
| Huile d'onagre poudre | 25 - 50 mg | |
| Spiruline poudre | 25 - 50 mg | |
| Citrate de zinc | 12 - 25 mg | |
| Alpha tocophérol | 3 - 6,50 mg | |
| Riboflavine | 0,40 - 0,80 mg | (vitamine B2) |
| Nicotinamide | 3 - 7 mg | (vitamine B3) |
| Pantothénate de calcium | 2 - 3,25 mg | (vitamine B5) |
| Chlorhydrate de pyridoxine | 0,5 - 1 mg | (vitamine B6) |
| Acide folique | 0,05 - 1 mg | (vitamine B9) |
| Cyanocobalamine | 0,00060 - 0,000120mg | (vitamine B12) |
| Sélénite de sodium | 0,042 - 0,084 mg | |
| Extrait de thé vert | 20 - 50 mg | |
| Extrait d'olive fruit | 20 - 35 mg | |
| Extrait de citrus | 10 - 25 mg | |
| Bêta carotène naturel | 10 - 20 mg | |
| Extrait de melon | 2 - 5 mg | |
| Extrait de tomate | 0,5 - 1 mg | |
| Picnogénol | 25-100 mg | |
| Xéaxantine | 2-8 mg | |

Pour ce comprimé particulier, la dose recommandée est de deux comprimés matin et soir.

## Revendications

1. Complément alimentaire, destiné à une administration par voie orale, comprenant une association :
(a) d'un mélange d'agents antioxydants et énergisants constitué des vitamines C et E, du co-enzyme Q10 et/ou une association de vitamines B2, B5, B6, B9, B 12 et C, du zinc, sous forme de sels ou de complexes, du glutathion ou de la lactoferrine, de la superoxyde dismutase, du sélénium et des acides gras insaturés ;
(b) d'un mélange d'agents énergisants constitué de la carnitine et/ou un produit capable de générer la carnitine par synthèse endogène, et du magnésium.

2. Complément alimentaire selon la revendication 1, **caractérisé en ce que** la source de carnitine est un extrait naturel, en particulier la spiruline.

3. Complément alimentaire selon l'une quelconque des revendications précédentes, **caractérisé en ce que** le produit capable de générer la carnitine par synthèse endogène est une association de vitamines B3 et C.

4. Complément alimentaire selon l'une quelconque des revendications précédentes, **caractérisé en ce que** les acides gras insaturés sont l'huile de bourrache et/ou l'huile d'onagre.

5. Complément alimentaire selon l'une quelconque des revendications précédentes, **caractérisé en ce que** la source du co-enzyme Q10 est l'huile de soja.

6. Complément alimentaire selon l'une quelconque des revendications précédentes, **caractérisé en ce que** le zinc est introduit sous la forme de citrate de zinc.

7. Complément alimentaire selon l'une quelconque des revendications précédentes, **caractérisé en ce que** le magnésium est introduit sous la forme d'oxyde de magnésium.

8. Complément alimentaire selon l'une quelconque des revendications précédentes, **caractérisé en ce que** le sélénium est introduit sous la forme de sélénite de sodium.

9. Complément alimentaire selon l'une quelconque des revendications précédentes, **caractérisé en ce que** la source de lactoferrine est un extrait de petit lait.

10. Complément alimentaire selon l'une quelconque des revendications précédentes, **caractérisé en ce que** la source de superoxyde dismutase est un extrait de melon.

11. Complément alimentaire selon l'une quelconque des revendications précédentes, **caractérisé en ce qu'**il comprend un troisième mélange (c) comprenant au moins un agent anti-oxydant choisi dans le groupe constitué par les catéchines, l'hydroxytyrosol, les flavonoïdes, la béta-carotène et leurs mélanges.

12. Complément alimentaire selon l'une quelconque des revendications précédentes, **caractérisé en ce qu'**il comprend un quatrième mélange (d) comprenant au moins un agent anti-oxydant choisi dans le groupe constitué par les OPC de pin et la xéaxantine.

13. Complément alimentaire selon l'une quelconque des revendications précédentes, **caractérisé en ce qu'**il comprend :
60 à 120 mg de vitamine C
3 à 6,5 mg de vitamine E
50 à 100 mg d'huile de soja
12 à 25 mg de citrate de zinc
50 à 100 mg d'extrait de petit lait
2 à 5 mg d'extrait de melon
0,042 à 0,084 mg de sélénite de sodium
25 à 50 mg d'huile de bourrache
25 à 50 mg d'huile d'onagre
25 à 50 mg de spiruline
3 à 7 mg de vitamine B3
80 à 120 mg d'oxyde de magnésium

14. Complément alimentaire selon l'une quelconque des revendications précédentes, **caractérisé en ce qu'**il comprend :
60 à 120 mg de vitamine C
3 à 6,5 mg de vitamine E
50 à 100 mg d'huile de soja
12 à 25 mg de citrate de zinc
50 à 100 mg d'extrait de petit lait
2 à 5 mg d'extrait de melon
0,042 à 0,084 mg de sélénite de sodium
25 à 50 mg d'huile de bourrache
25 à 50 mg d'huile d'onagre
25 à 50 mg de spiruline
3 à 7 mg de vitamine B3
80 à 120 mg d'oxyde de magnésium
25 à 100 mg de picnogénol
2 à 8 mg de Xéaxantine

15. Complément alimentaire selon l'une quelconque des revendications précédentes, pour son utilisation dans l'aide à la fertilité.

16. Complément alimentaire selon l'une quelconque des revendications précédentes, pour son utilisation dans la prévention des fausses couches, en particulier les fausses couches précoces.

17. Complément alimentaire selon l'une quelconque des revendications précédentes, pour son utilisation dans la prévention des malformations embryonnaires.

## Claims

1. Dietary supplement, intended for oral administration, comprising an association:
(a) of a mixture of antioxidant and energising agents consisting of vitamins C and E, coenzyme Q10 and/or an association of vitamins B2, B5, B6, B9, B12 and C, zinc, in salt or complex form, glutathione and lactoferrin, superoxide dismutase, selenium and unsaturated fatty acids;
(b) of a mixture of energising agents consisting of carnitine and/or a substance capable of generating carnitine by means of endogenous synthesis, and magnesium.

2. Dietary supplement according to claim 1, **characterised in that** the source of carnitine is a natural extract, particularly spirulina.

3. Dietary supplement according to the any of the above claims, **characterised in that** the substance capable of generating carnitine by means of endogenous synthesis is an association of vitamins B3 and C.

4. Dietary supplement according to any of the above claims, **characterised in that** the unsaturated fatty acids are borage oil and/or evening primrose oil.

5. Dietary supplement according to any of the above claims, **characterised in that** the source of coenzyme Q10 is soya oil.

6. Dietary supplement according to any of the above claims, **characterised in that** zinc is introduced in the form of zinc citrate.

7. Dietary supplement according to any of the above claims, **characterised in that** magnesium is introduced in the form of magnesium oxide.

8. Dietary supplement according to any of the above claims, **characterised in that** selenium is introduced in the form of sodium selenite.

9. Dietary supplement according to any of the above claims, **characterised in that** the source of lactoferrin is a whey extract.

10. Dietary supplement according to any of the above claims, **characterised in that** the source of superoxide dismutase is a melon extract.

11. Dietary supplement according to any of the above claims, **characterised in that** it comprises a third mixture (c) comprising at least one antioxidant agent selected from the group consisting of cathechins, hydroxythyrosol, flavonoids, beta-carotene and mixtures thereof.

12. Dietary supplement according to any of the above claims, **characterised in that** it comprises a fourth mixture (d) comprising at least one antioxidant agent selected from the group consisting of pine OPCs and zeaxanthin.

13. Dietary supplement according to any of the above claims, **characterised in that** it comprises:
60 to 120 mg of vitamin C
3 to 6.5 mg of vitamin E
50 to 100 mg of soya oil
12 to 25 mg of zinc citrate
50 to 100 mg of whey extract
2 to 5 mg of melon extract
0.042 to 0.084 mg of sodium selenite
25 to 50 mg of borage oil
25 to 50 mg of evening primrose oil
25 to 50 mg of spirulina
3 to 7 mg of vitamin B3
80 to 120 mg of magnesium oxide

14. Dietary supplement according to any of the above claims, **characterised in that** it comprises:
60 to 120 mg of vitamin C
3 to 6.5 mg of vitamin E
50 to 100 mg of soya oil
12 to 25 mg of zinc citrate
50 to 100 mg of whey extract
2 to 5 mg of melon extract
0.042 to 0.084 mg of sodium selenite
25 to 50 mg of borage oil
25 to 50 mg of evening primrose oil
25 to 50 mg of spirulina
3 to 7 mg of vitamin B3
80 to 120 mg of magnesium oxide
25 to 100 mg of picnogenol
2 to 8 mg of Zeaxanthin

15. Dietary supplement according to any of the above claims, for the use thereof in fertility assistance.

16. Dietary supplement according to any of the above claims, for the use thereof in the prevention of miscarriages, particularly early miscarriages.

17. Dietary supplement according to any of the above claims for the use thereof in the prevention of embryonic malformations.

## Patentansprüche

1. Nahrungsergänzungsmittel, das zur oralen Verabreichung bestimmt ist, umfassend eine Verbindung:
(a) aus einer Mischung von Antioxidationsmitteln und Energie spendenden Mitteln, die aus den Vitaminen C und E, dem Coenzym Q10 und/oder einer Verbindung aus den Vitaminen B2, B5, B6, B9, B12 und C, aus Zink in Form von Salzen oder Komplexen, Glutathion oder Lactoferrin, Superoxiddismutase, Selen und ungesättigten Fettsäuren besteht;
(b) aus einer Mischung von Energie spendenden Mitteln, die aus Carnitin und/oder einem Produkt, das imstande ist, Carnitin durch endogene Synthese zu erzeugen, und aus Magnesium besteht.

2. Nahrungsergänzungsmittel nach Anspruch 1, **dadurch gekennzeichnet, dass** die Carnitinquelle ein natürlicher Extrakt, insbesondere Spirulina, ist.

3. Nahrungsergänzungsmittel nach einem der vorhergehenden Ansprüche, **dadurch gekennzeichnet, dass** das Produkt, das imstande ist, Carnitin durch endogene Synthese zu erzeugen, eine Verbindung aus den Vitaminen B3 und C ist.

4. Nahrungsergänzungsmittel nach einem der vorhergehenden Ansprüche, **dadurch gekennzeichnet, dass** die ungesättigten Fettsäuren Borretschöl und/oder Nachtkerzenöl sind.

5. Nahrungsergänzungsmittel nach einem der vorhergehenden Ansprüche, **dadurch gekennzeichnet, dass** die Quelle des Coenzyms Q10 Sojaöl ist.

6. Nahrungsergänzungsmittel nach einem der vorhergehenden Ansprüche, **dadurch gekennzeichnet, dass** das Zink in Form von Zinkcitrat eingeleitet wird.

7. Nahrungsergänzungsmittel nach einem der vorhergehenden Ansprüche, **dadurch gekennzeichnet, dass** das Magnesium in Form von Magnesiumoxid eingeleitet wird.

8. Nahrungsergänzungsmittel nach einem der vorhergehenden Ansprüche, **dadurch gekennzeichnet, dass** das Selen in Form von Natriumselenit eingeleitet wird.

9. Nahrungsergänzungsmittel nach einem der vorhergehenden Ansprüche, **dadurch gekennzeichnet, dass** die Quelle des Lactoferrins ein Molkeextrakt ist.

10. Nahrungsergänzungsmittel nach einem der vorhergehenden Ansprüche, **dadurch gekennzeichnet, dass** die Quelle der Superoxiddismutase ein Melonenextrakt ist.

11. Nahrungsergänzungsmittel nach einem der vorhergehenden Ansprüche, **dadurch gekennzeichnet, dass** es eine dritte Mischung (c) umfasst, umfassend mindestens ein Antioxidationsmittel, das aus der Gruppe gewählt wird, die von Catechinen, Hydroxytyrosol, Flavonoiden, Beta-Carotin und deren Mischungen gebildet wird.

12. Nahrungsergänzungsmittel nach einem der vorhergehenden Ansprüche, **dadurch gekennzeichnet, dass** es eine vierte Mischung (d) umfasst, umfassend mindestens ein Antioxidationsmittel, das aus der Gruppe gewählt wird, die von OPC aus Pinien und Zeaxanthin gebildet wird.

13. Nahrungsergänzungsmittel nach einem der vorhergehenden Ansprüche, **dadurch gekennzeichnet, dass** es Folgendes umfasst:
60 bis 120 mg Vitamin C
3 bis 6,5 mg Vitamin E
50 bis 100 mg Sojaöl
12 bis 25 mg Zinkcitrat
50 bis 100 mg Molkeextrakt
2 bis 5 mg Melonenextrakt
0,042 bis 0,084 mg Natriumselenit
25 bis 50 mg Borretschöl
25 bis 50 mg Nachtkerzenöl
25 bis 50 mg Spirulina
3 bis 7 mg Vitamin B3
80 bis 120 mg Magnesiumoxid

14. Nahrungsergänzungsmittel nach einem der vorhergehenden Ansprüche, **dadurch gekennzeichnet, dass** es Folgendes umfasst:
60 bis 120 mg Vitamin C
3 bis 6,5 mg Vitamin E
50 bis 100 mg Sojaöl
12 bis 25 mg Zinkcitrat
50 bis 100 mg Molkeextrakt
2 bis 5 mg Melonenextrakt
0,042 bis 0,084 mg Natriumselenit
25 bis 50 mg Borretschöl
25 bis 50 mg Nachtkerzenöl
25 bis 50 mg Spirulina
3 bis 7 mg Vitamin B3
80 bis 120 mg Magnesiumoxid
25 bis 100 mg Pycnogenol
2 bis 8 mg Zeaxanthin

15. Nahrungsergänzungsmittel nach einem der vorhergehenden Ansprüche, für seine Verwendung zur Fruchtbarkeitsunterstützung.

16. Nahrungsergänzungsmittel nach einem der vorhergehenden Ansprüche, für seine Verwendung zur Vorbeugung gegen Fehlgeburten, insbesondere gegen frühzeitige Fehlgeburten.

17. Nahrungsergänzungsmittel nach einem der vorhergehenden Ansprüche, für seine Verwendung zur Vorbeugung gegen embryonale Fehlbildungen.
